Europäisches Patentamt
European Patent Office
Office européen des brevets

(19) (11) Publication number: **0 351 767 A2**

# EUROPEAN PATENT APPLICATION

(21) Application number: 89113117.9

(22) Date of filing: 18.07.89

(51) Int. Cl.4: A61K 31/44, A61K 31/40, A61K 31/00

The title of the invention has been amended (Guidelines for Examination in the EPO, A-III, 7.3).

(30) Priority: 18.07.88 US 220866

(43) Date of publication of application: 24.01.90 Bulletin 90/04

(84) Designated Contracting States: AT BE CH DE FR GB IT LI LU NL SE

(71) Applicant: E.R. SQUIBB & SONS, INC.
Lawrenceville-Princeton Road
Princeton New Jersey 08543-4000(US)

(72) Inventor: Grover, Gary James
Box 583, RD 1 Bowne Station Road
Stockton New Jersey 08559(US)
Inventor: Atwal, Karnail
92 Valley View Way
Newton, PA 18940(US)

(74) Representative: Staub, Gabriella, Dr.-Ing. et al
Baaderstrasse 3
D-8000 München 5(DE)

## (54) Use of potassium channel activators to inhibit myocardial cell necrosis and to maintain the functioning of the heart during myocardial ischemia and/or reperfusion.

(57) A method is provided for inhibiting myocardial cell necrosis and improving myocardial function during myocardial ischemia and/or reperfusion by administering a potent potassium channel activator such as pinacidil or cromakalim.

FIG. 1

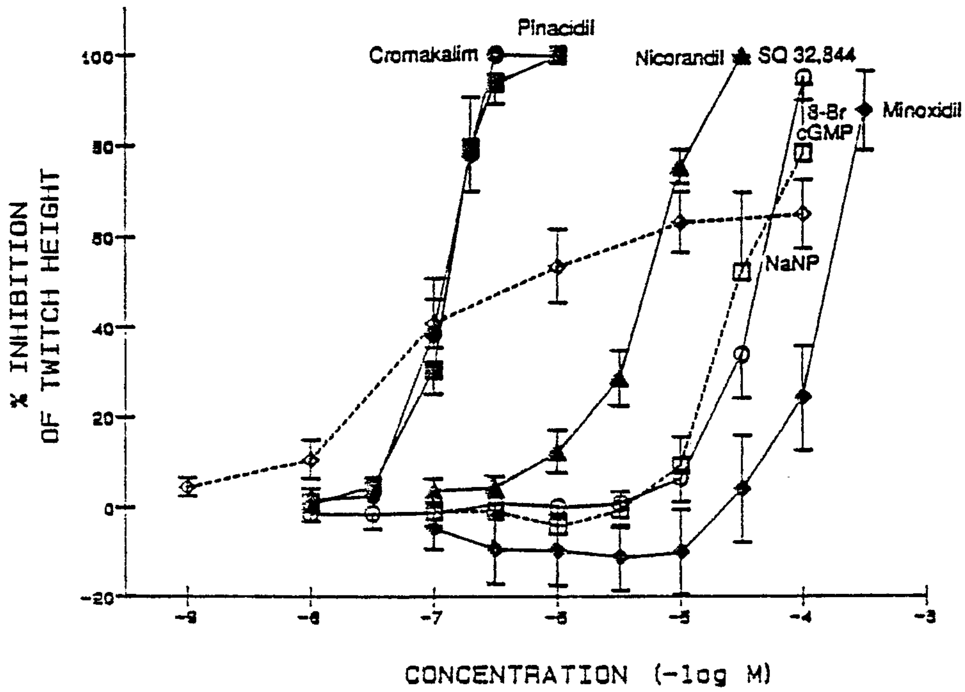


EP 0 351 767 A2

# METHOD FOR INHIBITING MYOCARDIAL CELL NECROSIS AND PRESERVING HEART FUNCTION DURING MYOCARDIAL ISCHEMIA AND/OR REPERFUSION

The present invention relates to a method for inhibiting myocardial cell necrosis and improving myocardial function in mammalian species during myocardial ischemia and/or reperfusion by administering a potent potassium channel activator to enhance the recovery of heart function.

Blood flow reductions in the heart can result in dysfunction of this organ and cell death if the flow reduction is severe enough. Restoration of coronary blood flow early during a heart attack is becoming a clinical reality with the advent and improvements in thrombolytic, mechanical, and surgical interventions. While early restoration of blood flow, for example, by thrombolysis or following transient ischemia, can prevent or mitigate the degree of cell death (infarction) occurring, reperfusion can still result in some degree of cardiac dysfunction or cell death (also referred to as stunned myocardia). Thus, it would be of great clinical value to find a means to preserve reperfusion function or cell viability of the heart.

Recently, a new class of compounds has been described and labeled potassium channel activators (PCA), Cook, N.S. "The pharmacology of potassium channels and their therapeutic potential," TIPS 9:21, 1988. Cook indicates "that the cellular site of action of a number of drugs used therapeutically seems to involve the modulation of membrane $K^+$ channels" and that "opening of $K^+$ channels by a new class of drugs appears to underlie their relaxation of a variety of smooth muscles" because of their ability to hyperpolarize and thus relax these cells.

Examples of such PCA drugs or compounds include (+)-2-cyano-1-(4-pyridyl)-3-(1,2,2-trimethylpropyl)-guanidine (hereinafter referred to as pinacidil) and (±)-6-cyano-3,4-dihydro-2,2-dimethyl-trans-4-(2-oxo-1-pyrrolidyl)-2H-benzo[b]-pyran-3-ol (hereinafter referred to as cromakalim or BRL 34915).

Other examples of compounds which are potassium channel activators but which have substantially weaker PCA activity include N-(2-hydroxyethyl)nicotinamide nitrate ester (hereinafter referred to as nicorandil) and 6-amino-1,2-dihydro-1-hydroxy-2-imino-4-piperidinopyrimidine (hereinafter referred to a minoxidil).

The above PCA compounds have been found to reduce blood pressure and thus may be useful as antihypertensive agents.

Imai, N., et al, "Comparative effects of nitroprusside and pinacidil on myocardial blood flow and infarct size in awake dogs with acute myocardial infarction." Circulation 77:705, 1988, discloses that pinacidil, a coronary vasodilator, has no effect on either blood flow to ischemic myocardium or infarct size. Gross, G. J., et al, "Comparative Effects of Nicorandil, Nitroglycerin, Nicotinic Acid and SG-86 on the Metabolic Status and Functional Recovery of the Ischemic-Reperfused Myocardium, J. Cardiovascular Pharmacology, 10 (Suppl. 8):S76-S84 (1987), disclose that "nicorandil and nicotinic acid infusion prior to and during a 15-min coronary occlusion resulted in a significantly improved recovery of mycocardial segment function (%SS, dL/dt) during a 3-h reperfusion period.... However, blood flow was more homogeneously distributed between ischemic and nonischemic regions following nicorandil treatment during reperfusion...." (page S83). It is believed that nicorandil's improvement of post-ischemic function is probably due to its nitrate-like action (for instance like nitroglycerin) and not due to its weak potency as a potassium channel activator (Taira, N., "Similarity and Dissimilarity in the Mode and Mechanism of Action between Nicorandil and Classical Nitrates: An Overview", J. Cardiovascular Pharmacology, 10 (Suppl. 8):S1-S9 (1987).

In accordance with the present invention, a method is provided for inhibiting myocardial cell necrosis and improving myocardial function in mammalian species during myocardial ischemia and/or reperfusion to improve heart function, wherein a therapeutically effective amount of a potent potassium channel activator is systemically administered, such as orally or parenterally, or by catheter, prior to, during or after reperfusion.

The term "reperfusion" is employed herein to refer to release of occlusion and resumption of blood flow.

It has been found that the potent potassium channel activator improves performance of the heart during and after myocardial ischemia when administered during both the coronary occlusion period and the reperfusion period or only during the reperfusion period. Such improvement in performance of the heart is evidenced by decreased contractile dysfunction and decrease in tissue necrosis (as measured by lactate dehydrogenase release).

Potassium channel activators which are suitable for use herein are those which exhibit potent inhibition of spontaneous mechanical activity in rat portal vein as described hereinafter, that is, an $IC_{50}$ (50% of the maximum inhibition) of less than 2 $\mu$M. Examples of such potassium channel activators suitable for use herein include cromakalim which has an $IC_{50}$ of about 0.12 $\mu$M and pinacidil which as an $IC_{50}$ of about 0.15 $\mu$M.

In carrying out the method of the present invention, the potassium channel activator may be administered to mammalian species, such as monkeys, dogs, cats, rats, humans, etc. during the period of coronary occlusion and/or during the period of reperfusion and/or shortly after termination of the ischemic attack, for example within 1 to 2 hours after the ischemia.

Although the potassium channel activator may be administered systemically, such as orally or parenterally, it is preferred that the potassium channel activator be administered locally to the coronary arteries by catheter such as by arterial angiography or intracoronary injection.

With regard to dosage of potassium channel activator, where the drug is administered by arterial angeography or intracoronary injection, from about 0.001 to about 30 mg/kg/treatment and preferably from about 0.5 to about 25 mg/kg/treatment will be employed. The number of treatments will depend upon the length of the ischemic attack and the progress of reperfusion to achieve normal heart function. Usually, from 1 to 5 treatments per day will be required for as long as contractile dysfunction continues.

Where the potassium channel activator is to be administered by angiography or intracoronary injection, it will be formulated in a conventional vehicle, such as distilled water, saline, Ringer's solution, or other conventional carriers.

The potassium channel activator may also be incorporated in a conventional dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

With regard to such systemic formulations, single or divided doses of from about 5 to about 2500 mg, preferably from about 10 to 2000 mg/one to four times daily, may be administered in systemic dosage forms as described above for a period sufficient to restore normal heart function.

Figure 1 is a graph of the cumulative concentration response curves for inhibition of spontaneous force development in rat portal veins;

Figure 2 is a graph of cumulative concentration response curves for affecting the frequency of spontaneous contractions;

Figure 3 are graphs of end diastolic pressure (EDP), reperfusion function and lactate dehydrogenase (LDH) release for each of vehicle, nicorandil and pinacidil; and

Figure 4 are graphs of lactate dehydrogenase (LDH) release and reperfusion function for each of vehicle and cromakalim (BRL 34915) at concentrations of 1 $\mu$M and 7 $\mu$M.

## Example A

## Design of In Vitro Screen for Identification of "Potent" Potassium Channel Activators Suitable for Use Herein

### Methods

Solutions:

A bicarbonate buffered physiological salt solution (PSS) was used containing, in mM: 118.4 NaCl, 4.7 KCl, 1.2 $KH_2PO_4$ 1.2 $MgSO_4$, 2.5 $CaCl_2$, 25.0 $NaHCO_3$ and 11.7 glucose. PSS with varying concentrations of KCl was prepared by addition of appropriate amounts of KCl from a 4M KCl stock. Calcium free PSS was prepared by deletion of $CaCl_2$ and addition of 1.0 mM [ethylenebis(oxyethylenitrilo)]tetraacetic acid (EGTA). All solutions were aerated with 95% $O_2$-5% $CO_2$ such that the pH was 7.4. Experiments were conducted at 37° C.

Stock solutions of test compounds were prepared daily. The compounds were dissolved in water or DMSO as appropriate.

Tissue Preparation:

Normotensive male Wistar Kyoto rats approximately 14 weeks old were sacrificed using $CO_2$. The

thoracic aorta and portal vein were quickly removed and placed into cold PSS. Blood was rinsed from the lumen and adherent connective tissue was carefully removed. Rings approximately 4 mm in width were cut from each aorta. The endothelium was mechanically removed by inserting a wire into the lumen and gently rolling the ring on filter paper moistened with PSS. The aortic rings were mounted on stainless steel tissue wires attached to tissue holders. An intact segment of portal vein extending approximately 2 cm from the liver hilus was mounted longitudinally on tissue holders. The tissues were suspended in individual 20 ml organ chambers and connected to a micrometer for control of tissue length and a Grass FT.03 force transducer. Mechanical responses were recorded on Grass Model 7D polygraphs.

The following compounds were tested.

Table I.

| Primary mechanisms of action of vasodilators. | |
|---|---|
| Mechanism of Action | Compounds Tested |
| Potassium channel activation | Chromakalim (BRL 34915), pinacidil, nicorandil, minoxidil, minoxidil sulfate, SQ 32,844 (trans)-4-amino-3,4-dihydro-3-hydroxy-2,2-dimethyl-2H-1-benzopyran-6-carbonitrile |
| Guanylate cyclase activator | Na nitroprusside, nicorandil, 8-Br cyclic GMP |

Experimental Protocol:

The rat portal vein segments were gradually stretched over a 1 to 2 hour equilibration period to a preload of 0.5 g. When the frequency and height of the spontaneous twitches had stabilized, propranolol (1 μM) was added and cumulative concentration response curves were obtained for the test compounds. A steady state reduction of the twitch height and frequency was reached before the next concentration was added.

Evaluation of Results:

Force development by the portal vein segments was determined at the peak of the twitch. The effect of each compound on force was calculated relative to the force in its absence. $IC_{50}$ values were determined at the concentration where 50% of the maximal inhibition force was attained. In addition, the relative frequency of twitches was determined by counting the number of twitches in a 5 minute period following drug addition and determining the change in rate from basal (absence of test compund). Results were then calculated as % inhibition of twitch frequency. Data are report as mean ± SEM.

Results:

Responses in rat portal vein:

The potassium channel activators inhibited the spontaneous mechanical response in rat portal vein in a concentration dependent manner (Table I and Figure 1). The spontaneous mechanical activity in the portal vein was slightly more sensitive to cromakalim and pinacidil than were the phenylephrine contractions in the aorta. The portal vein was less sensitive to nicorandil and sodium nitroprusside. Additionally, sodium nitroprusside did not completely inhibit the twitch height even at concentrations up to 100 μM. The sensitivity of response to 8-bromo cyclic GMP and minoxidil and the cromakalin metabolite SQ 32,844 was similar in the two tissues.

Table II.

| Inhibition of spontaneous mechanical activity in rat portal vein by various vasodilators. | |
|---|---|
| Compound | $IC_{50}$ (μM)[a] |
| Cromakalim | 0.12 |
| Pinacidil | 0.15 |
| Nicorandil | 5.1 |
| Minoxidil | 150 |
| SQ 32,844 | 38 |
| Sodium nitroprusside | 0.042[b] |
| 8-Bromo cyclic GMP | 43 |

[a]Mean ± SEM of four cumulative concentration response curves for all agonists except cromakalim and pinacidil (N = 5).
[b]$IC_{50}$ taken at 50% of the maximum inhibition attained.

The test compounds, with the exception of sodium nitroprusside, reduced the frequency of twitches in a concentration dependent manner at approximately the same concentrations needed to reduce twitch height. Sodium nitroprusside increased twitch frequency with increasing concentration.

The spontaneous mechanical activity of the portal vein is thought to result from opening of voltage dependent calcium channels in response to depolarization associated with pacemakers. Thus, the portal vein provides a good model to investigate the influence of the hyperpolarization by the potassium channel activators on the voltage dependent channels. The inhibitory effects of the potassium channel activators involves a dose dependent reduction in twitch height with a concomitant reduction in twitch frequency. The decrease in force may be due to the hyperpolarizing influence of the potassium channel activators counteracting the depolarizing effect of the pacemakers, thereby leading to an effective blockade of the voltage dependent calcium channels. The hyperpolarization could also increase the time needed for the smooth muscle membranes to reach threshold, thus reducing the twitch frequency. Cromakalim, pinacidil, minoxidil, and SQ 32,844 exhibited similar potencies in the aorta and portal vein. The reduced potency of nicorandil, a potassium channel activator with nitrate properties, and sodium nitroprusside may indicate that guanylate cyclase plays a lesser role in relaxation of the portal vein than the aorta. Interestingly, sodium nitroprusside actually caused an increase in twitch frequency with the reduction in twitch height. 8-Bromo cyclic GMP, which bypasses the activation of guanylate cyclase, exhibited similar potency in the two blood vessels and reduced twitch frequency and height.

Conclusion: The inhibition of twitch height is an index of the potassium channel activating activity of the compounds tested.

Figure 1 shows cumulative concentration response curves for inhibition of spontaneous force development in rat portal veins. Cromakalin (●) and pinacidil (■) were essentially equipotent followed by nicorandil (▲), 8-bromo cyclic GMP (□), SQ 32,844 (O), and minoxidil (♦). Sodium nitroprusside (◊) was incapable of inhibiting force development by more than 60% even at concentrations of 100 μM. Figure 2 shows cumulative concentration response curves for affecting the frequency of the spontaneous contractions. Symbols are the same as for Figure 1. With the exception of sodium nitroprusside, all the compounds decreased the frequency of the contractions in a concentration related fashion. Data are plotted as mean ± SEM of 4 veins from individual rats.

For purposes of the present invention, potent potassium channel activators which may be employed in the method of the invention include cromakalim and pinacidil which have an $IC_{50}$ of less than 2μM in the inhibition of spontaneous mechanical activity in rat portal vein and exclude nicorandil and minoxidil which have an $IC_{50}$ of 5.1 μM and 150 μM, respectively.

The following working Examples represent preferred embodiments of the present invention.

## Example 1

An injectable solution for use in administering pinacidil by intracoronary injection, by arterial angiography or intravenously is produced as follows:

| | |
|---|---|
| Pinacidil | 50 mg |
| Methyl paraben | 5 mg |
| Propyl paraben | 1 mg |
| Sodium chloride | 25 g |
| Water for injection qs. | 5 l. |

The pinacidil, preservatives and sodium chloride are dissolved in 3 liters of water for injection and then the volume is brought up to 5 liters. The solution is filtered through a sterile filter and aseptically filled into presterilized vials which are then closed with presterilized rubber closures. Eaoh vial contains a concentration of 75 mg of active ingredient per 150 ml of solution.

The above injectable is suitable for use in inhibiting myocardial necrosis and improving heart function during coronary occlusion and when administered during coronary occlusion and/or the reperfusion period.

## Example 2

An injectable for use in inhibiting myocardial cell necrosis and improving myocardial function during ischemia and/or reperfusion is prepared as described in Example 1 except that the potassium channel activator employed is cromakalim.

## Example 3

A pinacidil formulation for use in inhibiting myocardial cell necrosis and improving myocardial function during myocardial ischemia and reperfusion suitable for oral administration is set out below.

1000 tablets each containing 50 mg of pinacidil were produced from the following ingredients.

| | |
|---|---|
| Pinacidil | 50 g |
| Corn starch | 50 g |
| Gelatin | 7.5 g |
| Avicel (microcrystalline cellulose) | 25 g |
| Magnesium stearate | 2.5 g |

The pinacidil and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 50 mg of active ingredient.

## Example 4

A tablet formulation for use in inhibiting myocardial cell necrosis and improving myocardial function during myocardial ischemia and reperfusion is prepared as described in Example 3 except that cromakalim is employed in place of pinacidil.

## Example 5

The following experiment was conducted to determine the effect of pinacidil, cromakalim and nicorandil on myocardial cell necrosis and heart function during myocardial ischemia and reperfusion.

## Method

For the determination of the effect of the potassium channel activators pinacidil and BRL 34915 (cromakalim) on post-ischemic necrosis and function, isolated, buffer perfused rat hearts were used. Male Sprague-Dawely rats (450-550 g) were used for all experiments. The rats were anesthetized using 30 mg/kg sodium pentobarbital (i.p.) They were intubated and then treated with i.v. heparin (1000 U/kg). While being mechanically ventilated, their hearts were perfused in situ via retrogade cannulation of the aorta. The hearts were then excised and quickly moved to a Langendorff apparatus where they were perfused with Krebs-Henseleit (1.25 mM $CaCl_2$) buffer at a constant pressure (80 mmHg). A saline-filled latex balloon attached to a metal cannula was then inserted into the left ventricle and connected to a Statham pressure transducer for measurement of left ventricular systolic and developed (LVDP) pressure. The hearts were allowed to equilibrate for 15 minutes at which time end diastolic pressure (EDP) was adjusted to 5 mm Hg and this was maintained for 5 minutes. Pre-ischemia, pre-drug function (HRXLVDP/1000), heart rate, and coronary blood flow (extracorporeal electromagnetic flow probe, Carolina Medical Electronics, King, N.C.) were then determined.

Once the baseline measurements were made, the hearts were divided into 4 groups (n = 4-6 each): 1. Hearts receiving vehicle. 2. Hearts receiving 1, 10 or 100 µM pinacidil. 3. Hearts receiving 1 or 7 µM BRL 34915. 4. Hearts receiving 1, 10 or 100 µM nicroandil. Each heart received its respective treatment in the perfusion buffer for 10 minutes at which time functional and flow parameters were again measured. All hearts were then made globally ischemic by shutting off the perfusate for 25 minutes. Reperfusion was then instituted with the normal nondrug treated buffer. Reperfusion was maintained for 30 minutes at which time flow and functional parameters were again measured. Lactate dehydrogenase (LDH) release into the reperfusion effluent was measured and this is a measure of myocardial cell death or necrosis. The severity of ischemia was determined by the return of function during reperfusion, mitigation of contracture (lower end diastolic pressure) and LDH release.

## Results

All of the compounds used have been shown to be potassium channel activators and have a ranked order of potency as follows: BRL 34915 > pinacidil > nicorandil. This is shown on Figures 1 and 2 where spontaneously contracting guinea pig portal veins are relaxed by the various potassium activators of interest. The inhibition of twitch height is an index of the potassium channel activating activity of these compounds.

At 1 and 100 µM concentration, pinacidil did not show any marked anti-ischemic activity and at the 100 µM dose, a tendency for toxic effects (before ischemia reduction in function) appeared. At the 10 µM concentration, pinacidil significantly improved post-ischemic cardiac function and reduced reperfusion end diastolic pressure (EDP) (contracture). These data are shown on Figure 3. At the doses used, pinacidil did not significantly reduce necrosis as measured by lactate dehydrogenase (LDH) release, however, it showed a tendency to do so. Nicroandil was not showed to be effective at any dose used and the data for 10 µM nicorandil are shown on Figure 3. On Figure 4, similar data are shown for 7 µM cromakalim (BRL 34915). In this case, myocardial function was improved during reperfusion and LDH release and thus necrosis were reduced. Thus, the potent potassium channel activators pinacidil and cromakalim can reduce the severity of ischemia and improve post-ischemic recovery of function. This is important in patients undergoing coronary artery bypass and graft procedures as well as other methods of coronary recanalization. This is also important in patients undergoing episodes of transient ischemia as in many types of angina pectoris.

## Claims

1. A potent potassium channel activator for use in reducing or eliminating myocardial cell necrosis, ischemia injury and/or reperfusion injury in mammalian species.

2. The potent potassium channel activator as defined in claim 1 for use by arterial angiography or by intracoronary injection, intravenously or orally.

3. The potent potassium channel activator as defined in claim 1 for use by administration prior to during or after reperfusion.

4. The potent potassium channel activator according to claim 1 for use by administration during coronary occlusion or ischemia and reperfusion.

5. The potent potassium channel activator according to claim 1 for use by administration only during reperfusion.

6. The potent potassium channel activator according to claim 1 which exhibits potent inhibition of spontaneous mechanical activity in rat portal vein and has an $IC_{50}$ of less than about 2 μM.

7. The potent potassium channel activator according to claim 1 which is pinacidil or cromakalim.

FIG. 1

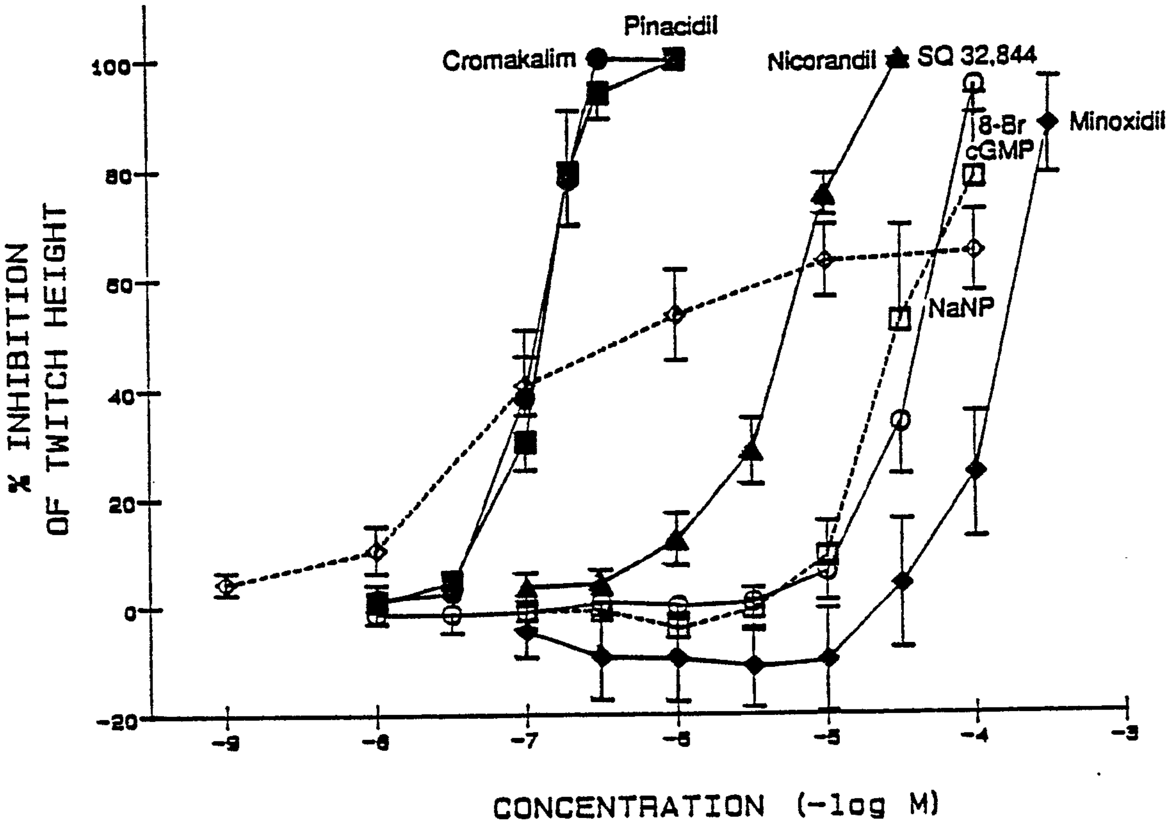
Pinacidil
Cromakalim
Nicorandil
SQ 32,844
8-Br cGMP
Minoxidil
NaNP
100
80
60
40
20
0
-20
% INHIBITION OF TWITCH HEIGHT
-9
-8
-7
-6
-5
-4
-3
CONCENTRATION (-log M)

FIG. 2

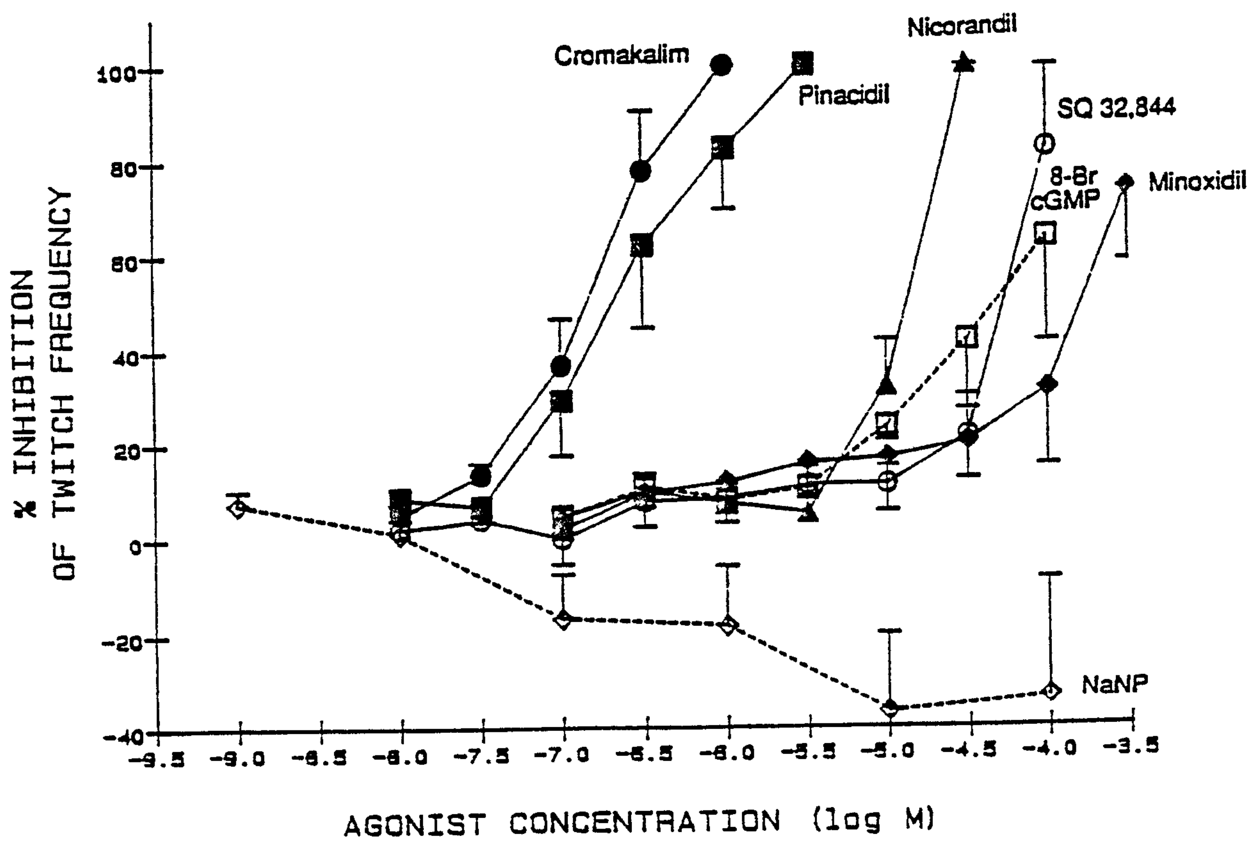
Nicorandil
Cromakalim
Pinacidil
SQ 32,844
8-Br cGMP
Minoxidil
NaNP
100
80
60
40
20
0
-20
-40
% INHIBITION OF TWITCH FREQUENCY
-9.5
-9.0
-8.5
-8.0
-7.5
-7.0
-6.5
-6.0
-5.5
-5.0
-4.5
-4.0
-3.5
AGONIST CONCENTRATION (log M)

FIG. 3

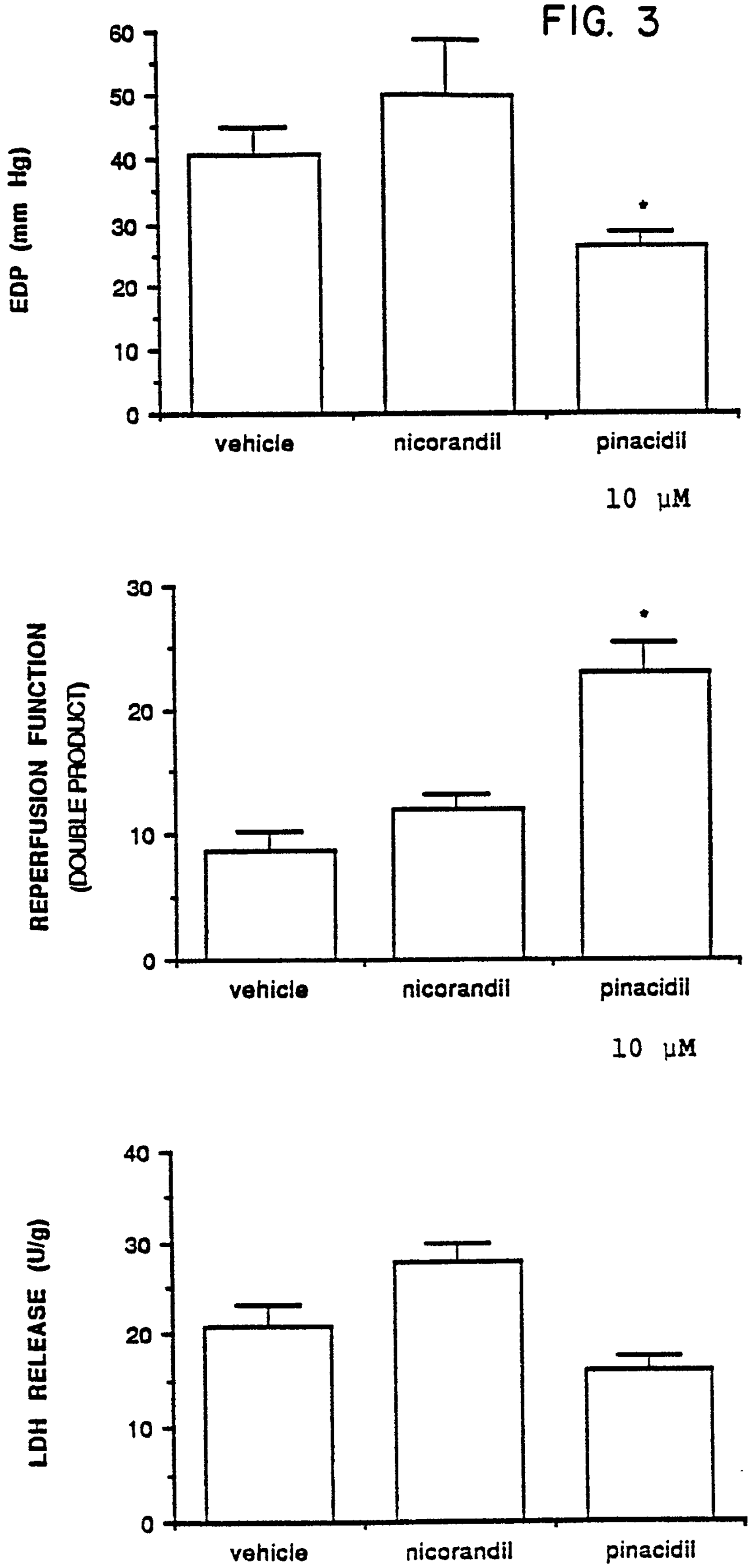
EDP (mm Hg)
0
10
20
30
40
50
60
*
vehicle
nicorandil
pinacidil
10 μM
REPERFUSION FUNCTION
(DOUBLE PRODUCT)
0
10
20
30
*
vehicle
nicorandil
pinacidil
10 μM
LDH RELEASE (U/g)
0
10
20
30
40
vehicle
nicorandil
pinacidil

FIG. 4

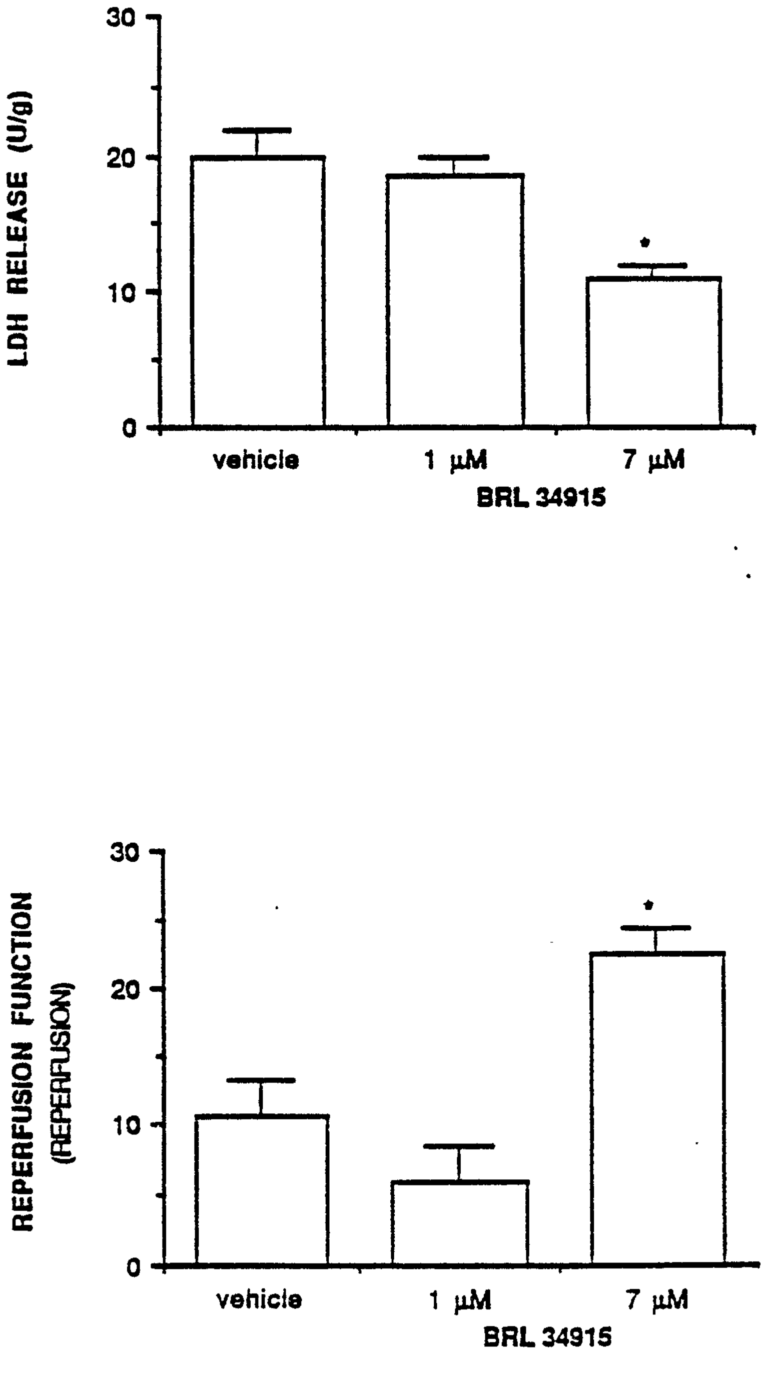
30
20
10
0
LDH RELEASE (U/g)
*
vehicle
1 μM
7 μM
BRL 34915
30
20
10
0
REPERFUSION FUNCTION
(REPERFUSION)
*
vehicle
1 μM
7 μM
BRL 34915